# FASCICULE DE BREVET EUROPEEN

(11) **EP 3 606 898 B1**
(45) Date de publication et mention de la délivrance du brevet: **02.10.2024**
(21) Numéro de dépôt: 18729153.9
(22) Date de dépôt: 12.06.2018
(51) Int. Cl.: C07C 39/06, C07C 39/08

(54) **COMPOSITIONS COMPRENANT DE L'HYDROQUINONE ET DU CATECHOL, PROCEDE DE PREPARATION DE CES COMPOSITIONS**
ZUSAMMENSETZUNGEN, HYDROCHINON UND CATECHOL UMFASSEND, VERFAHREN ZUR HERSTELLUNG DIESER ZUSAMMENSETZUNGEN
COMPOSITIONS COMPRISING HYDROQUINONE AND CATECHOL, METHOD FOR PREPARING THESE COMPOSITIONS

(30) Priorité: 13.06.2017 FR 1755272
(43) Date de publication de la demande: 12.02.2020
(73) Titulaire: SPECIALTY OPERATIONS FRANCE, 69003 Lyon (FR)
(72) Inventeur: GAREL, Laurent, 69003 Lyon (FR)
(74) Mandataire: Valentino, Cédric
(86) Numéro de dépôt international: PCT/EP2018/065463
(87) Numéro de publication internationale: WO 2018/229033

(56) Documents cités:
- WO-A1-98/02500
- US-A- 5 426 244
- US-A- 5 679 223

## Description

### DOMAINE TECHNIQUE

La présente invention concerne des compositions comprenant de l'hydroquinone et/ou du catéchol, ainsi qu'un procédé de préparation de ces compositions par hydroxylation du phénol.

### ART ANTERIEUR

Les composés aromatiques hydroxylés sont des composés importants en synthèse organique. Différentes voies de synthèse de ces produits ont été développées au cours du temps en particulier par hydroxylation du phénol en présence d'un catalyseur. La réaction d'hydroxylation du phénol conduit à l'obtention de deux isomères à savoir le 1,4-dihydroxybenzène ou hydroquinone (HQ) et le 1,2-dihydroxybenzène ou catéchol (PC) qui sont des composés à fort potentiel industriel. L'hydroquinone est un produit utilisé dans de nombreux domaines d'application en tant qu'inhibiteur de polymérisation, antioxydant dans les élastomères ou comme intermédiaire de synthèse. Un autre domaine d'application est la photographie. Le catéchol est quant à lui un produit largement utilisé notamment comme intermédiaire dans la synthèse de molécules utilisables dans des applications variées telles que la pharmacie, l'agrochimie, la parfumerie ou l'industrie alimentaire. On peut citer le document WO 1998/02500 qui décrit des compositions comprenant au moins un dérivé du catéchol, un solvant aromatique et un alcool. Ces compositions sont utilisées en tant qu'inhibiteurs de polymérisation. Ces compositions restent liquides à des températures allant jusqu'à -50°C et permettent ainsi d'éviter l'utilisation de lignes industrielles thermostatées. Le document US 5679223 décrit un procédé de purification d'un mélange comprenant catéchol, 3-méthylcatéchol et 4-méthylcatéchol.

Compte-tenu de ce large champ d'exploitation, il est nécessaire de produire ces produits à une échelle industrielle et de disposer de procédés de fabrication optimisés.

Classiquement, l'hydroquinone et le catéchol sont produits par hydroxylation du phénol par le peroxyde d'hydrogène, en présence d'un catalyseur acide, acide protique fort (par exemple dans FR 2 071 464) ou bien un catalyseur solide à propriétés acides comme par exemple la zéolithe TS-1 (FR 2 489 816), une zéolithe de type silicalite de titane MEL (EP 1 131 264), une titanozéolithe de type MFI (EP 1 123 159) ou encore une zéolithe MCM-22 (FR 2 856 681).

L'un des enjeux de ces procédés est d'optimiser de façon générale la productivité de la réaction pour pouvoir satisfaire à la demande en hydroquinone et en catéchol. Les paramètres optimisés pourront être notamment les rendements de la réaction, le ratio entre l'hydroquinone et le catéchol, ou l'efficacité énergétique de la réaction.

Pour répondre à ce problème général de productivité, de nombreux documents font état de conditions de réactions spécifiques. Par exemple, la nature du solvant ou des solvants utilisés pour la réaction est décrite dans la publication scientifique de Thangaraj et al., Indian Journal of Chemistry, Vol.33A, March 1994, pp.255-258, ainsi que dans la demande de brevet EP 3 048 091 et dans le brevet US5426244.

A l'issue de telle réaction d'hydroxylation, on obtient généralement un mélange comprenant de l'hydroquinone et du catéchol, en proportions variables, ainsi que différents sous-produits tels que du résorcinol, du pyrogallol, etc. A la connaissance des inventeurs, la formation de sous-produits indésirables dérivés du solvant ou des solvants n'a jamais été étudiée, dans le cadre d'une réaction d'hydroxylation du phénol. Or, la présence de ces sous-produits peut avoir une influence sur l'efficacité de la réaction elle-même, mais aussi sur les procédés de purifications subséquents.

### BREVE DESCRIPTION DE L'INVENTION

Il est du mérite de la société demanderesse d'avoir identifié une nouvelle composition d'hydroquinone et/ou de catéchol contenant certains sous-produits dont la nature et les taux sont maîtrisés.

Ainsi, un premier objet de la présente invention est une composition comprenant au moins un composé choisi parmi l'hydroquinone et le catéchol, caractérisée en ce qu'elle comprend en outre entre 0,1 et 10 000 ppm d'au moins un composé choisi parmi 2-(alkoxy)phénol, 4-(alkoxy)phénol, 2-(alkyl)phénol, 4-(alkyl)phénol, (alkyl)catéchol, (alkyl)hydroquinone et alkoxybenzène, dans lequel le groupement alkoxy est choisi parmi tert-butoxy, et isopropoxy et le groupement alkyl est choisi parmi tert-butyl et isopropyl.

Préférentiellement, un objet de la présente invention est une composition comprenant au moins un composé choisi parmi l'hydroquinone et le catéchol, caractérisée en ce qu'elle comprend en outre entre 0,1 et 10 000 ppm d'au moins un composé choisi parmi 2-(*tert-*butoxy)phénol, 4-(tert-butoxy)phénol, 2-(tert-butyl)phénol, 4-(tert-butyl)phénol, (*tert-*butyl)catéchol, (tert-butyl)hydroquinone et tert-butoxybenzène.

Selon un autre aspect, un objet de la présente invention est une composition comprenant au moins un composé choisi parmi l'hydroquinone et le catéchol, caractérisée en ce qu'elle comprend en outre entre 0,1 et 10 000 ppm d'au moins un composé choisi parmi 2-(isopropoxy)phénol, 4-(isopropoxy)phénol, 2-isopropylphénol, 4-isopropylphénol, isopropylcatéchol, isopropylhydroquinone et isopropylbenzène.

Un autre aspect de cette invention concerne un procédé de préparation d'une composition comprenant au moins un composé choisi parmi hydroquinone et catéchol selon l'invention caractérisé en ce qu'il comprend une étape (a) de réaction du phénol avec de l'eau oxygénée en présence d'un catalyseur, dans un solvant comprenant un alcool, ou un mélange d'alcools, choisi parmi l'isopropanol, le 2,2-diméthylpropanol et le tert-butanol.

### DESCRIPTION DETAILLEE

Dans le cadre de la présente invention, et sauf indication contraire, l'expression « compris entre ... et... » inclut les bornes. Sauf indications contraires, les pourcentages et ppm sont des pourcentages et ppm massiques.

Dans le cadre de la présente invention, et sauf indication contraire, l'expression « alkyl » représente une chaine linéaire ou branchée comprenant de 1 à 6 atomes de carbone.

Dans le cadre de la présente invention, et sauf indication contraire, l'expression « alkoxy » représente un groupe alkyle lié à un atome d'oxygène : R-O.

Dans le cadre de la présente invention, et sauf indication contraire, le terme « ppm » signifie « partie par million ». Cette unité représente une fraction massique : 1 ppm = 1 mg/kg.

Un premier aspect de la présente invention est une composition comprenant au moins un composé choisi parmi l'hydroquinone et le catéchol, caractérisée en ce qu'elle comprend en outre entre 0,1 et 10 000 ppm d'au moins un composé choisi parmi 2-(alkoxy)phénol, 4-(alkoxy)phénol, 2-(alkyl)phénol, 4-(alkyl)phénol, (alkyl)catéchol, (alkyl)hydroquinone et alkoxybenzène, dans lequel le groupement alkoxy est choisi parmi tert-butoxy, et isopropoxy et le groupement alkyl est choisi parmi tert-butyl et isopropyl.

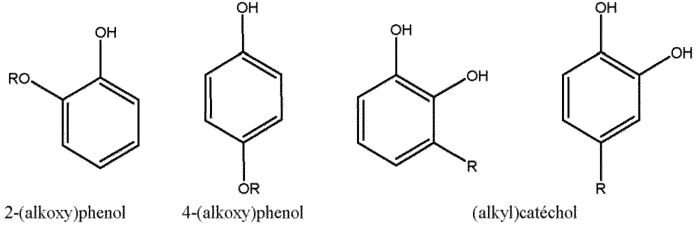

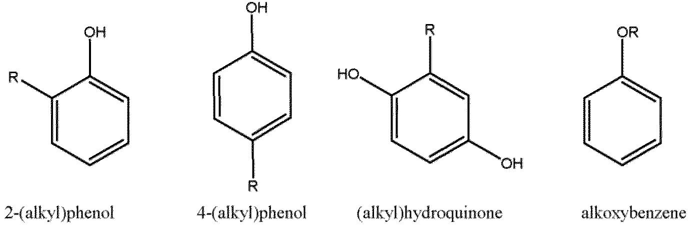

Préférentiellement, la composition comprenant au moins un composé choisi parmi l'hydroquinone et le catéchol est caractérisée en ce qu'elle comprend en outre au moins un composé choisi parmi 2-(alkoxy)phénol, 4-(alkoxy)phénol, 2-(alkyl)phénol, 4-(alkyl)phénol, (alkyl)catéchol et (alkyl)hydroquinone en une quantité :
- supérieure ou égale à 0,5 ppm, préférentiellement une quantité supérieure ou égale à 1 ppm et encore plus préférentiellement une quantité supérieure ou égale à 10 ppm ; et/ou
- inférieure ou égale à 500 ppm, préférentiellement une quantité inférieure ou égale à 300 ppm et encore plus préférentiellement une quantité inférieure ou égale à 100 ppm.

En outre, la composition selon la présente invention peut comprendre une quantité supérieure ou égale à 1 ppm, préférentiellement une quantité supérieure ou égale à 10 ppm et encore plus préférentiellement une quantité supérieure ou égale à 100 ppm d'alkoxybenzène. La composition selon la présente invention peut en outre une quantité inférieure ou égale à 10 000 ppm, préférentiellement une quantité inférieure ou égale à 5 000 ppm et encore plus préférentiellement une quantité inférieure ou égale à 1 000 ppm de alkoxybenzène.

Selon un aspect particulier, la composition selon la présente invention ne comprend pas de solvant aromatique.

Dans le cadre de la présente invention, il est aussi possible que des dérivés de types dialkoxylés, dialkylés ou alkylés et alkoxylés soient formés lors de la réaction. On peut citer, de manière non limitative, des dérivés du type 2,3-dialkoxyphénol, 2,6-dialkoxyphénol, 2,3-dialkylphénol, 2,6-dialkylphénol, 2-alkoxy-6-alkylphénol ou 2-alkoxy-3-alkylphénol.

Avantageusement, un objet de la présente invention concerne une composition comprenant au moins un composé choisi parmi l'hydroquinone et le catéchol, caractérisée en ce qu'elle comprend en outre entre 0,1 et 10 000 ppm d'au moins un composé choisi parmi 2-(*tert*-butoxy)phénol, 4-(*tert*-butoxy)phénol, 2-(*tert*-butyl)phénol, *4-(tert-*butyl)phénol, (*tert*-butyl)catéchol, (tert-butyl)hydroquinone et *tert*-butoxybenzène. 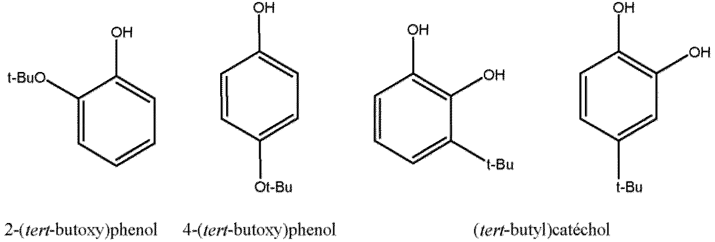 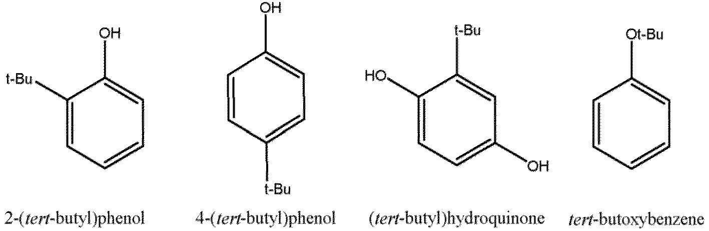 Préférentiellement, la composition comprenant au moins un composé choisi parmi l'hydroquinone et le catéchol est caractérisée en ce qu'elle comprend en outre au moins un composé choisi parmi 2-(*tert*-butoxy)phénol, 4-(*tert*-butoxy)phénol, 2-(*tert*-butyl)phénol, 4-(*tert*-butyl)phénol, (*tert*-butyl)catéchol et (*tert*-butyl)hydroquinone en une quantité :
- supérieure ou égale à 0,5 ppm, préférentiellement une quantité supérieure ou égale à 1 ppm et encore plus préférentiellement une quantité supérieure ou égale à 10 ppm ; et/ou
- inférieure ou égale à 500 ppm, préférentiellement une quantité inférieure ou égale à 300 ppm et encore plus préférentiellement une quantité inférieure ou égale à 100 ppm.

En outre, la composition selon la présente invention peut comprendre une quantité supérieure ou égale à 1 ppm, préférentiellement une quantité supérieure ou égale à 10 ppm et encore plus préférentiellement une quantité supérieure ou égale à 100 ppm de *tert-*butoxybenzène. La composition selon la présente invention peut en outre une quantité inférieure ou égale à 10 000 ppm, préférentiellement une quantité inférieure ou égale à 5 000 ppm et encore plus préférentiellement une quantité inférieure ou égale à 1 000 ppm de *tert-*butoxybenzène.

Avantageusement, un objet de la présente invention concerne une composition comprenant au moins un composé choisi parmi l'hydroquinone et le catéchol, caractérisée en ce qu'elle comprend en outre entre 0,1 et 10 000 ppm d'au moins un composé choisi parmi 2-(isopropoxy)phénol, 4-(isopropoxy)phénol, 2-isopropylphénol, 4-isopropylphénol, isopropylcatéchol, isopropylhydroquinone et isopropylbenzène. 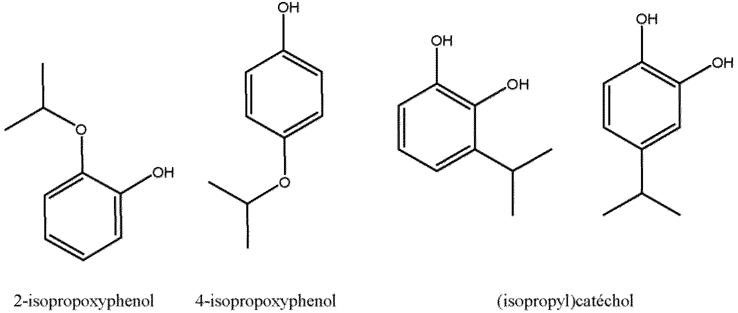 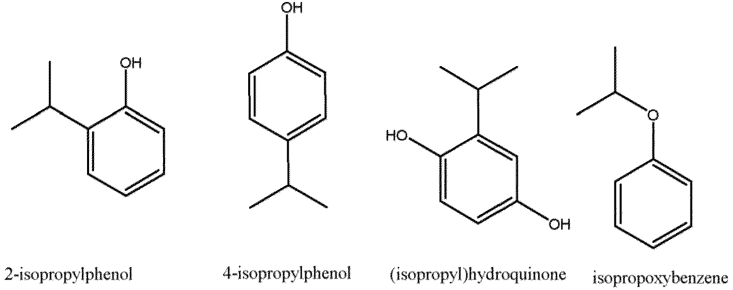 Préférentiellement, la composition comprenant au moins un composé choisi parmi l'hydroquinone et le catéchol est caractérisée en ce qu'elle comprend en outre au moins un composé choisi parmi 2-(isopropoxy)phénol, 4-(isopropoxy)phénol, 2-isopropylphénol, 4-isopropylphénol, isopropylcatéchol, isopropylhydroquinone en une quantité :
- supérieure ou égale à 0,5 ppm, préférentiellement une quantité supérieure ou égale à 1 ppm et encore plus préférentiellement une quantité supérieure ou égale à 10 ppm ; et/ou
- inférieure ou égale à 500 ppm, préférentiellement une quantité inférieure ou égale à 300 ppm et encore plus préférentiellement une quantité inférieure ou égale à 100 ppm.

En outre, la composition selon la présente invention peut comprendre une quantité supérieure ou égale à 1 ppm, préférentiellement une quantité supérieure ou égale à 10 ppm et encore plus préférentiellement une quantité supérieure ou égale à 100 ppm de isopropylbenzène. La composition selon la présente invention peut en outre une quantité inférieure ou égale à 10 000 ppm, préférentiellement une quantité inférieure ou égale à 5 000 ppm et encore plus préférentiellement une quantité inférieure ou égale à 1 000 ppm de isopropylbenzène.

Un aspect de la présente invention se rapporte à une composition comprenant :
- au moins 80% d'hydroquinone, préférentiellement au moins 90% d'hydroquinone, plus préférentiellement au moins 99% d'hydroquinone, et
- une quantité supérieure ou égale à 0,1 ppm, préférentiellement une quantité supérieure ou égale à 1 ppm et encore plus préférentiellement une quantité supérieure ou égale à 10 ppm d'au moins un composé choisi parmi 2-(alkoxy)phénol, 4-(alkoxy)phénol, (alkyl)catéchol et (alkyl)hydroquinone.

Avantageusement, la quantité en au moins un composé choisi parmi 2-(alkoxy)phénol, 4-(alkoxy)phénol, (alkyl)catéchol et (alkyl)hydroquinone peut être inférieure ou égale à 1 000 ppm, préférentiellement inférieure ou égale à 500 ppm et encore plus préférentiellement inférieure ou égale à 100 ppm.

Selon un autre aspect, l'invention se rapporte à une composition comprenant :
- au moins 80% de catéchol, préférentiellement au moins 90% de catéchol, plus préférentiellement au moins 99% de catéchol, et
- une quantité supérieure ou égale à 0,1 ppm, préférentiellement une quantité supérieure ou égale à 1 ppm et encore plus préférentiellement une quantité supérieure ou égale à 10 ppm d'au moins un composé choisi parmi 2-(alkyl)phénol et 4-(alkyl)phénol.

Avantageusement, la quantité en au moins un composé choisi parmi 2-(alkyl)phénol et 4-(alkyl)phénol peut être inférieure ou égale à 1 000 ppm, préférentiellement inférieure ou égale à 500 ppm et encore plus préférentiellement ou égale à 100 ppm.

Avantageusement, la présente invention se rapporte à une composition comprenant :
- au moins 80% d'hydroquinone, préférentiellement au moins 90% d'hydroquinone, plus préférentiellement au moins 99% d'hydroquinone, et
- une quantité supérieure ou égale à 0,1 ppm, préférentiellement une quantité supérieure ou égale à 1 ppm et encore plus préférentiellement une quantité supérieure ou égale à 10 ppm d'au moins un composé choisi parmi 2-(*tert*-butoxy)phénol, 4-(*tert-*butoxy)phénol, (*tert*-butyl)catéchol et (*tert*-butyl)hydroquinone.

Avantageusement, la quantité en au moins un composé choisi parmi 2-(*tert-*butoxy)phénol, 4-(*tert*-butoxy)phénol, (*tert*-butyl)catéchol et (*tert*-butyl)hydroquinone peut être inférieure ou égale à 1 000 ppm, préférentiellement inférieure ou égale à 500 ppm et encore plus préférentiellement inférieure ou égale à 100 ppm.

Selon un autre aspect, l'invention se rapporte à une composition comprenant :
- au moins 80% de catéchol, préférentiellement au moins 90% de catéchol, plus préférentiellement au moins 99% de catéchol, et
- une quantité supérieure ou égale à 0,1 ppm, préférentiellement une quantité supérieure ou égale à 1 ppm et encore plus préférentiellement une quantité supérieure ou égale à 10 ppm d'au moins un composé choisi parmi 2-(*tert-*butyl)phénol et 4-(*tert-*butyl)phénol.

Avantageusement, la quantité en au moins un composé choisi parmi 2-(*tert-*butyl)phénol et 4-(*tert*-butyl)phénol peut être inférieure ou égale à 1 000 ppm, préférentiellement inférieure ou égale à 500 ppm et encore plus préférentiellement ou égale à 100 ppm.

Avantageusement, la présente invention se rapporte à une composition comprenant :
- au moins 80% d'hydroquinone, préférentiellement au moins 90% d'hydroquinone, plus préférentiellement au moins 99% d'hydroquinone, et
- une quantité supérieure ou égale à 0,1 ppm, préférentiellement une quantité supérieure ou égale à 1 ppm et encore plus préférentiellement une quantité supérieure ou égale à 10 ppm d'au moins un composé choisi parmi 2-(isopropoxy)phénol, 4-(isopropoxy)phénol, isopropylcatéchol, isopropylhydroquinone.

Avantageusement, la quantité en au moins un composé choisi parmi 2-(isopropoxy)phénol, 4-(isopropoxy)phénol, isopropylcatéchol, isopropylhydroquinone peut être inférieure ou égale à 1 000 ppm, préférentiellement inférieure ou égale à 500 ppm et encore plus préférentiellement inférieure ou égale à 100 ppm.

Selon un autre aspect, l'invention se rapporte à une composition comprenant :
- au moins 80% de catéchol, préférentiellement au moins 90% de catéchol, plus préférentiellement au moins 99% de catéchol, et
- une quantité supérieure ou égale à 0,1 ppm, préférentiellement une quantité supérieure ou égale à 1 ppm et encore plus préférentiellement une quantité supérieure ou égale à 10 ppm d'au moins un composé choisi parmi 2-isopropylphénol, 4-isopropylphénol.

Avantageusement, la quantité en au moins un composé choisi parmi 2-isopropylphénol, 4-isopropylphénol peut être inférieure ou égale à 1 000 ppm, préférentiellement inférieure ou égale à 500 ppm et encore plus préférentiellement ou égale à 100 ppm.

Les compositions objet de la présente invention sont particulièrement intéressantes car elles répondent à un impératif de maitrise d'un procédé industriel. La présence et la teneur des sous-produits mentionnés sont compatibles d'une part avec un procédé d'hydroxylation du phénol de haute productivité offrant une large fenêtre opératoire de production de PC et de HQ, et d'autre part avec les procédés de purification, ce qui permet de maîtriser les coûts globaux du procédé.

Un autre aspect de la présente invention concerne un procédé de préparation d'une composition comprenant au moins un composé choisi parmi hydroquinone et catéchol selon l'invention caractérisé en ce qu'il comprend une étape (a) de réaction du phénol avec de l'eau oxygénée en présence d'un catalyseur, dans un solvant comprenant un alcool, ou un mélange d'alcools, choisi parmi l'isopropanol, le 2,2-diméthylpropanol et le *tert*-butanol.

L'étape (a) correspond à une hydroxylation du phénol. Ce procédé permet la synthèse d'hydroquinone (HQ) et de catéchol (PC). Avantageusement le procédé selon l'invention permet de maîtriser le ratio PC/HQ. Le ratio molaire PC/HQ est inférieur ou égal à 1, de manière préférée inférieur ou égal à 0,7.

L'eau oxygénée (H₂O₂) utilisée dans la présente invention peut être en solution dans l'eau. N'importe quelle dilution d'H₂O₂ dans l'eau peut être utilisée. Une concentration usuelle de 30% dans l'eau peut être utilisée dans le cadre de ce procédé, mais une solution aqueuse présentant une concentration plus élevée dans l'eau peut aussi être utilisée telle quelle ou sous forme diluée avec un solvant inerte qui peut être sélectionné parmi les alcools, de préférence méthanol, éthanol, isopropanol, butanol, tert-butanol. L'eau oxygénée peut être ajoutée en une seule fois. L'eau oxygénée peut également être ajoutée par portion dans la réaction sur une période de temps donné.

Selon le procédé de la présente invention, la réaction est réalisée en présence d'un catalyseur, de préférence un catalyseur hétérogène. Le catalyseur peut être une zéolite comprenant du titane. Ces zéolites peuvent présenter une structure de la famille MFI ou de la famille MEL. Le catalyseur peut être une titanosilicalite. La composition de la titanosilicalite selon la présente invention peut être représentée par la structure suivante : (SiO₂)ₓ(TiO₂)₍₁₋ₓ₎. La valeur de x/(1-x) peut être comprise entre 5 et 1000, de préférence entre 10 et 500. La titanosilicalite peut être préparée selon des méthodes connues de l'homme du métier, on peut citer par exemple le procédé du document US 4,410,501. Le catalyseur peut être de préférence une TS-1, TS-2, Ti-MWW, Ti-MCM68, de manière plus préférée le catalyseur est une TS-1.

La quantité de catalyseur utilisé dans la présente invention est en général supérieure ou égale à 0,1% en masse par rapport au phénol, de préférence supérieure ou égale à 0,4% en masse. La quantité de catalyseur utilisé dans la présente invention est en général inférieure ou égale à 30% en masse, de préférence inférieure ou égale à 20% en masse.

Dans le procédé selon l'invention, le solvant comprend un alcool, ou un mélange d'alcools, l'alcool est choisi parmi l'isopropanol, le 2,2-diméthylpropanol et le *tert*-butanol. Ledit alcool peut être utilisé seul ou avec un co-solvant. Le co-solvant peut avantageusement être choisi parmi l'eau.

Avantageusement le ratio massique entre l'alcool utilisé dans la réaction et le co-solvant peut être compris entre 1:99 et 90:10, de préférence compris entre 3:97 et 80:20.

La quantité d'alcool utilisé représente entre 1 et 90 % en poids, de préférence entre 3 et 50% en poids par rapport à la masse totale de liquide dans la réaction.

La quantité d'eau utilisée peut être la quantité d'eau apportée par la solution aqueuse d'eau oxygénée. La quantité de co-solvant représente entre 5 et 90% en poids, de préférence entre 8 et 90% en poids, de manière plus préférentielle entre 8 et 85% en poids par rapport à la masse totale de liquide dans la réaction.

Préférentiellement, dans le procédé selon l'invention, le solvant comprend du *tert-*butanol. Le *tert*-butanol peut être utilisé seul ou avec un co-solvant. Le co-solvant peut avantageusement être choisi parmi l'eau, l'acétone, l'acétonitrile, le 1,4-dioxane ou un alcool de formule R'-OH, ou leurs mélanges, où R' représente une chaine alkyl linéaire ou branchée comprenant de 1 à 6 atomes de carbones, de préférence l'alcool est choisi parmi le méthanol, l'éthanol, l'isopropanol, le n-propanol, n-butanol. Avantageusement le ratio massique entre le tert-butanol utilisé dans la réaction et le co-solvant peut être compris entre 1:99 et 90:10, de préférence compris entre 3:97 et 80:20.

La quantité de *tert*-butanol utilisé représente entre 1 et 90 % en poids, de préférence entre 3 et 50% en poids par rapport à la masse totale de liquide dans la réaction.

La quantité d'eau utilisée peut être la quantité d'eau apportée par la solution aqueuse d'eau oxygénée. La quantité de co-solvant représente entre 5 et 90% en poids, de préférence entre 8 et 90% en poids, de manière plus préférentielle entre 8 et 85% en poids par rapport à la masse totale de liquide dans la réaction.

La température de la réaction peut être supérieure ou égale à 30°C, de préférence supérieure ou égale à 40°C. La température de la réaction peut être inférieure ou égale à 130°C, de préférence inférieure ou égale à 100°C.

Ce procédé peut être réalisé en continu, semi-continu, ou en batch.

A l'issue de l'étape (a), on peut obtenir avantageusement la composition selon l'invention comprenant au moins un composé choisi parmi l'hydroquinone et le catéchol, caractérisée en ce qu'elle comprend en outre entre 0,1 et 10 000 ppm d'au moins un composé choisi parmi 2-(alkoxy)phénol, 4-(alkoxy)phénol, 2-(alkyl)phénol, 4-(alkyl)phénol, (alkyl)catéchol, (alkyl)hydroquinone et alkoxybenzène.

Préférentiellement, à l'issue de l'étape (a), on peut obtenir avantageusement la composition selon l'invention comprenant au moins un composé choisi parmi l'hydroquinone et le catéchol, caractérisée en ce qu'elle comprend en outre entre 0,1 et 10 000 ppm d'au moins un composé choisi parmi 2-(*tert*-butoxy)phénol, 4-(*tert-*butoxy)phénol, 2-(*tert*-butyl)phénol, 4-(*tert*-butyl)phénol, (*tert*-butyl)catéchol, (*tert-*butyl)hydroquinone et *tert*-butoxybenzène.

Préférentiellement, à l'issue de l'étape (a), on peut obtenir avantageusement la composition selon l'invention comprenant au moins un composé choisi parmi l'hydroquinone et le catéchol, caractérisée en ce qu'elle comprend en outre entre 0,1 et 10 000 ppm d'au moins un composé choisi parmi 2-(isopropoxy)phénol, 4-(isopropoxy)phénol, 2-isopropylphénol, 4-isopropylphénol, isopropylcatéchol, isopropylhydroquinone et isopropylbenzène.

Selon un mode de réalisation de la présente invention, le procédé décrit ci-dessus peut en outre comprendre une étape (b) de purification de la composition obtenue à l'étape (a). Le choix de la méthode de purification n'est pas particulièrement limité. La méthode de purification utilisée peut notamment être choisie parmi l'extraction liquide/liquide, la distillation, la cristallisation, ou une combinaison de ces méthodes.

Selon un autre mode de réalisation de la présente invention, le procédé selon l'invention peut comprendre en outre une étape (c) de séparation de l'hydroquinone et du catéchol.

Ainsi à l'issue de l'étape (c), on peut obtenir :
- d'une part une composition comprenant au moins 80% d'hydroquinone et entre 0,1 et 1 000 ppm d'au moins un composé choisi parmi 2-(alkoxy)phénol, 4-(alkoxy)phénol, (alkyl)catéchol et (alkyl)hydroquinone.
- et d'autre part une composition comprenant au moins 80% de catéchol et entre 0,1 et 1 000 ppm d'au moins un composé choisi parmi 2-(alkyl)phénol et 4-(alkyl)phénol.

Préférentiellement, à l'issue de l'étape (c), on peut obtenir :
- d'une part une composition comprenant au moins 80% d'hydroquinone et entre 0,1 et 1 000 ppm d'au moins un composé choisi parmi 2-(*tert*-butoxy)phénol, 4-(*tert-*butoxy)phénol, (*tert*-butyl)catéchol et (*tert*-butyl)hydroquinone.
- et d'autre part une composition comprenant au moins 80% de catéchol et entre 0,1 et 1 000 ppm d'au moins un composé choisi parmi 2-(*tert*-butyl)phénol et 4-(*tert-*butyl)phénol.

Préférentiellement, à l'issue de l'étape (c), on peut obtenir :
- d'une part une composition comprenant au moins 80% d'hydroquinone et entre 0,1 et 1 000 ppm d'au moins un composé choisi parmi 2-(isopropoxy)phénol, 4-(isopropoxy)phénol, isopropylcatéchol et isopropylhydroquinone.
- et d'autre part une composition comprenant au moins 80% de catéchol et entre 0,1 et 1 000 ppm d'au moins un composé choisi parmi 2-isopropylphénol et 4-isopropylphénol.

Eventuellement, le procédé selon la présente invention peut comprendre au moins une étape (d) de mise en forme des produits obtenus à l'étape (c). Le choix de la méthode de mise en forme des produits obtenus à l'issue de l'étape (c) n'est pas limité. Les produits obtenus selon le procédé de l'invention peuvent ainsi être cristallisés ou sous forme de poudre amorphe. Les produits obtenus selon le procédé de l'invention peuvent être mis sous forme d'écailles, de perles, de billes, de pastilles, amorphes ou cristallisées.

## Revendications

1. Composition comprenant au moins un composé choisi parmi l'hydroquinone et le catéchol, **caractérisée en ce qu'**elle comprend en outre entre 0,1 et 10 000 ppm d'au moins un composé choisi parmi 2-(alkoxy)phénol, 4-(alkoxy)phénol, 2-(alkyl)phénol, 4-(alkyl)phénol, (alkyl)catéchol, (alkyl)hydroquinone et alkoxybenzène dans lequel le groupement alkoxy est choisi parmi tert-butoxy, et isopropoxy et le groupement alkyl est choisi parmi tert-butyl et isopropyl.

2. Composition selon la revendication 1 **caractérisée en ce qu'**elle comprend en outre au moins un composé choisi parmi 2-(alkoxy)phénol, 4-(alkoxy)phénol, 2-(alkyl)phénol, 4-(alkyl)phénol, (alkyl)catéchol et (alkyl)hydroquinone en une quantité :
- supérieure ou égale à 0,5 ppm, préférentiellement une quantité supérieure ou égale à 1 ppm et encore plus préférentiellement une quantité supérieure ou égale à 10 ppm ; et/ou
- inférieure ou égale à 500 ppm, préférentiellement une quantité inférieure ou égale à 300 ppm et encore plus préférentiellement une quantité inférieure ou égale à 100 ppm.

3. Composition selon l'une quelconque des revendications 1 à 2, comprenant au moins 80% d'hydroquinone et entre 0,1 et 1 000 ppm d'au moins un composé choisi parmi 2-(alkoxy)phénol, 4-(alkoxy)phénol, (alkyl)catéchol et (alkyl)hydroquinone.

4. Composition selon l'une quelconque des revendications 1 à 3, comprenant au moins 80% de catéchol et entre 0,1 et 1 000 ppm d'au moins un composé choisi parmi 2-(alkyl)phénol et 4-(alkyl)phénol.

5. Composition selon la revendication 1 comprenant au moins un composé choisi parmi l'hydroquinone et le catéchol, **caractérisée en ce qu'**elle comprend en outre entre 0,1 et 10 000 ppm d'au moins un composé choisi parmi 2-(*tert*-butoxy)phénol, 4*-*(*tert-*butoxy)phénol, 2-(*tert*-butyl)phénol, 4-(*tert*-butyl)phénol, (*tert*-butyl)catéchol, (*tert-*butyl)hydroquinone et *tert*-butoxybenzène.

6. Composition selon la revendication 1 comprenant au moins un composé choisi parmi l'hydroquinone et le catéchol, **caractérisée en ce qu'**elle comprend en outre entre 0,1 et 10 000 ppm d'au moins un composé choisi 2-(isopropoxy)phénol, 4-(isopropoxy)phénol, 2-isopropylphénol, 4-isopropylphénol, isopropylcatéchol, isopropylhydroquinone et isopropylbenzène.

7. Procédé de préparation d'une composition comprenant au moins un composé choisi parmi hydroquinone et catéchol définie selon l'une quelconque des revendications 1 à 6 **caractérisé en ce qu'**il comprend une étape (a) de réaction du phénol avec de l'eau oxygénée en présence d'un catalyseur, dans un solvant comprenant un alcool, ou un mélange d'alcools **caractérisé en ce que** le solvant est choisi parmi l'isopropanol, le 2,2-diméthylpropanol et le *tert*-butanol.

8. Procédé selon l'une quelconque des revendications 7 **caractérisé en ce qu'**il comprend en outre au moins une étape (b) de purification de la composition obtenue à l'étape (a).

9. Procédé selon la revendication 8 **caractérisé en ce qu'**il comprend en outre une étape (c) de séparation de l'hydroquinone et du catéchol, éventuellement, au moins une étape (d) de mise en forme des produits purifiés obtenus à l'étape (c), les produits obtenus à l'étape (d) pouvant être cristallisés ou sous forme de poudre amorphe.

10. Procédé selon l'une quelconque des revendications 7 à 9 **caractérisé en ce que** le catalyseur est un catalyseur hétérogène, préférentiellement une zéolite comprenant du titane, de préférence une TS-1, TS-2, Ti-MWW, Ti-MCM68, plus préférentiellement une TS-1.

11. Procédé selon l'une quelconque des revendications 7 à 10 **caractérisé en ce que** le solvant comprend un alcool, ou un mélange d'alcools, préférentiellement choisi parmi l'isopropanol, le 2,2-diméthylpropanol et le *tert*-butanol, en présence d'un co-solvant choisi parmi l'eau et un alcool choisi parmi méthanol, éthanol, isopropanol, n-propanol, n-butanol.

## Patentansprüche

1. Zusammensetzung, die mindestens eine aus Hydrochinon und Brenzcatechin ausgewählte Verbindung umfasst, **dadurch gekennzeichnet, dass** sie außerdem zwischen 0,1 und 10000 ppm mindestens einer Verbindung umfasst, die aus einem 2-(Alkoxy)phenol, 4-(Alkoxy)phenol, 2-(Alkyl)phenol, 4-(Alkyl)phenol, (Alkyl)brenzcatechin, (Alkyl)hydrochinon und Alkoxybenzol ausgewählt ist, wobei die Alkoxygruppe aus tert-Butoxy und Isopropoxy ausgewählt ist und die Alkylgruppe aus tert-Butyl und Isopropyl ausgewählt ist.

2. Verbindung nach Anspruch 1, **dadurch gekennzeichnet, dass** sie außerdem mindestens eine aus einem 2-(Alkoxy)phenol, 4-(Alkoxy)phenol, 2-(Alkyl)phenol, 4-(Alkyl)phenol, (Alkyl)brenzcatechin und (Alkyl)hydrochinon ausgewählte Verbindung mit einer Menge:
- von mehr als oder gleich 0,5 ppm, vorzugsweise mit einer Menge von mehr als oder gleich 1 ppm und noch stärker bevorzugt mit einer Menge von mehr als oder gleich 10 ppm und/oder
- von weniger als oder gleich 500 ppm, vorzugsweise mit einer Menge von weniger als oder gleich 300 ppm und noch stärker bevorzugt mit einer Menge von weniger als oder gleich 100 ppm umfasst.

3. Zusammensetzung nach einem der Ansprüche 1 bis 2, die mindestens 80 % Hydrochinon und zwischen 0,1 und 1000 ppm mindestens einer aus einem 2-(Alkoxy)phenol, 4-(Alkoxy)phenol, (Alkyl)brenzcatechin und (Alkyl)hydrochinon ausgewählten Verbindung umfasst.

4. Zusammensetzung nach einem der Ansprüche 1 bis 3, die mindestens 80 % Brenzcatechin und zwischen 0,1 und 1000 ppm mindestens einer aus einem 2-(Alkyl)phenol und 4-(Alkyl)phenol ausgewählten Verbindung umfasst.

5. Zusammensetzung nach Anspruch 1, die mindestens eine aus Hydrochinon und Brenzcatechin ausgewählte Verbindung umfasst, **dadurch gekennzeichnet, dass** sie außerdem zwischen 0,1 und 10000 ppm mindestens einer aus 2-(tert-Butoxy)phenol, 4-(tert-Butoxy)phenol, 2-(tert-Butyl)phenol, 4-(tert-Butyl)phenol, (tert-Butyl)brenzcatechin, (tert-Butyl)hydrochinon und tert-Butoxybenzol ausgewählten Verbindung umfasst.

6. Zusammensetzung nach Anspruch 1, die mindestens eine aus Hydrochinon und Brenzcatechin ausgewählte Verbindung umfasst, **dadurch gekennzeichnet, dass** sie außerdem zwischen 0,1 und 10000 ppm mindestens einer aus 2-(Isopropoxy)phenol, 4-(Isopropoxy)phenol, 2-Isopropylphenol, 4-Isopropylphenol, Isopropylbrenzcatechin, Isopropylhydrochinon und Isopropylbenzol ausgewählten Verbindung umfasst.

7. Verfahren zur Herstellung einer Zusammensetzung, die mindestens eine aus Hydrochinon und Brenzcatechin ausgewählte Verbindung gemäß der Definition in einem der Ansprüche 1 bis 6 umfasst, **dadurch gekennzeichnet, dass** es einen Schritt (a) der Umsetzung von Phenol mit Wasserstoffperoxid in Gegenwart eines Katalysators in einem einen Alkohol oder eine Alkoholmischung umfassenden Lösungsmittel umfasst, **dadurch gekennzeichnet, dass** das Lösungsmittel aus Isopropanol, 2,2-Dimethylpropanol und tert-Butanol ausgewählt ist.

8. Verfahren nach Anspruch 7, **dadurch gekennzeichnet, dass** es außerdem mindestens einen Schritt (b) der Reinigung der in Schritt (a) erhaltenen Zusammensetzung umfasst.

9. Verfahren nach Anspruch 8, **dadurch gekennzeichnet, dass** es außerdem einen Schritt (c) der Abtrennung von Hydrochinon und Brenzcatechin, gegebenenfalls mindestens einen Schritt (d) des Formens der in Schritt (c) erhaltenen gereinigten Produkte umfasst, wobei die in Schritt (d) erhaltenen Produkte kristallisiert oder in Form eines amorphen Pulvers vorliegen können.

10. Verfahren nach einem der Ansprüche 7 bis 9, **dadurch gekennzeichnet, dass** es sich beim Katalysator um einen heterogenen Katalysator, vorzugsweise einen Titan enthaltenden Katalysator, vorzugsweise ein TS-1, TS-2, Ti-MWW, Ti-MCM68, stärker bevorzugt ein TS-1, handelt.

11. Verfahren nach einem der Ansprüche 7 bis 10, **dadurch gekennzeichnet, dass** das Lösungsmittel einen Alkohol oder eine Mischung von Alkoholen, der bzw. die aus Isopropanol, 2,2-Dimethylpropanol und tert-Butanol ausgewählt ist bzw. sind, in Gegenwart eines Hilfslösungsmittels, das aus Wasser und einem aus Methanol, Ethanol, Isopropanol, n-Propanol, n-Butanol ausgewählten Alkohol ausgewählt ist, umfasst.

## Claims

1. Composition comprising at least one compound selected from hydroquinone and catechol, **characterized in that** it additionally comprises between 0.1 and 10 000 ppm of at least one compound selected from 2-(alkoxy)phenol, 4-(alkoxy)phenol, 2-(alkyl)phenol, 4-(alkyl)phenol, (alkyl)catechol, (alkyl)hydroquinone and alkoxybenzene in which the alkoxy group is selected from tert-butoxy and isopropoxy and the alkyl group is selected from tert-butyl and isopropyl.

2. Composition according to Claim 1, **characterized in that** it additionally comprises at least one compound selected from 2-(alkoxy)phenol, 4-(alkoxy)phenol, 2-(alkyl)phenol, 4-(alkyl)phenol, (alkyl)catechol and (alkyl)hydroquinone in an amount:
- of greater than or equal to 0.5 ppm, preferably an amount of greater than or equal to 1 ppm and more preferably still an amount of greater than or equal to 10 ppm; and/or
- of less than or equal to 500 ppm, preferably an amount of less than or equal to 300 ppm and more preferably still an amount of less than or equal to 100 ppm.

3. Composition according to either one of Claims 1 and 2, comprising at least 80% of hydroquinone and between 0.1 and 1000 ppm of at least one compound selected from 2-(alkoxy)phenol, 4-(alkoxy)phenol, (alkyl)catechol and (alkyl)hydroquinone.

4. Composition according to any one of Claims 1 to 3, comprising at least 80% of catechol and between 0.1 and 1000 ppm of at least one compound selected from 2-(alkyl)phenol and 4-(alkyl)phenol.

5. Composition according to Claim 1, comprising at least one compound selected from hydroquinone and catechol, **characterized in that** it additionally comprises between 0.1 and 10 000 ppm of at least one compound selected from 2-(tert-butoxy)phenol, 4-(tert-butoxy)phenol, 2-(tert-butyl)phenol, 4-(tert-butyl)phenol, (tert-butyl)catechol, (tert-butyl)hydroquinone and tert-butoxybenzene.

6. Composition according to Claim 1, comprising at least one compound selected from hydroquinone and catechol, **characterized in that** it additionally comprises between 0.1 and 10 000 ppm of at least one compound selected from 2-(isopropoxy)phenol, 4-(isopropoxy)phenol, 2-isopropylphenol, 4-isopropylphenol, isopropylcatechol, isopropylhydroquinone and isopropylbenzene.

7. Process for the preparation of a composition comprising at least one compound selected from hydroquinone and catechol defined according to any one of Claims 1 to 6, **characterized in that** it comprises a step (a) of reaction of phenol with hydrogen peroxide in the presence of a catalyst, in a solvent comprising an alcohol, or a mixture of alcohols, **characterized in that** the solvent is selected from isopropanol, 2,2-dimethylpropanol and tert-butanol.

8. Process according to Claim 7, **characterized in that** it additionally comprises at least one step (b) of purification of the composition obtained in step (a).

9. Process according to Claim 8, **characterized in that** it additionally comprises a step (c) of separation of the hydroquinone and catechol, optionally at least one step (d) of shaping the purified products obtained in step (c), it being possible for the products obtained in step (d) to be crystalline or in amorphous powder form.

10. Process according to any one of Claims 7 to 9, **characterized in that** the catalyst is a heterogeneous catalyst, preferably a titanium-comprising zeolite, preferably a TS-1, TS-2, Ti-MWW or Ti-MCM68, more preferably a TS-1.

11. Process according to any one of Claims 7 to 10, **characterized in that** the solvent comprises an alcohol, or a mixture of alcohols, preferably selected from isopropanol, 2,2-dimethylpropanol and tert-butanol, in the presence of a cosolvent selected from water and an alcohol selected from methanol, ethanol, isopropanol, n-propanol and n-butanol.
